# EUROPÄISCHE PATENTSCHRIFT

(11) **EP 3 320 865 B1**
(45) Veröffentlichungstag und Bekanntmachung des Hinweises auf die Patenterteilung: **19.08.2020**
(21) Anmeldenummer: 16197862.2
(22) Anmeldetag: 09.11.2016
(51) Int. Cl.: A61B 17/3203

(54) **HANDSTÜCK ZUM AUFSTRAHLEN EINES FLÜSSIGKEITSSTRAHLS UND EINSETZTEIL FÜR DIESES HANDSTÜCK**
HANDPIECE FOR SPRAYING A FLUIDJET AND INSERT PIECE FOR THIS HAND PIECE
PIÈCE À MAIN DESTINÉE À PULVÉRISER UN LIQUIDE ET ÉLÉMENT D'INJECTION POUR LADITE PIÈCE À MAIN

(43) Veröffentlichungstag der Anmeldung: 16.05.2018
(73) Patentinhaber: MEDAXIS AG, 6340 Baar (CH)
(72) Erfinder: Moser, Beat, 8926 Uerzlikon (CH); Widmer, Beat, 6005 Luzern (CH); Bütler, Martin, 6276 Hohenrain (CH); Good, Roman, 8057 Zürich (CH); Röthlin, Cyrill, 6331 Hünenberg (CH)
(74) Vertreter: Grünecker Patent- und Rechtsanwälte PartG mbB

(56) Entgegenhaltungen:
- EP-A1- 2 251 142
- DE-A1- 4 120 613
- US-A- 1 393 562
- US-A- 2 395 479
- US-A- 3 705 693
- US-A1- 2015 209 936

## Beschreibung

Die vorliegende Erfindung betrifft ein Handstück zum Aufstrahlen eines Fluidstrahls mit einem Handstückgehäuse, in dem ein Düsenelement zum Ausbilden der Strahlgeometrie des Fluidstrahls aufgenommen ist.

Ein solches Handstück ist beispielsweise aus der EP 2 251 142 A1 bekannt.

Die ausführlich diskutierte DE 41 20 613 A1 offenbart ein Düsenelement und einen Düsenträger. Eine als Überwurfmutter ausgebildete Düsenhaube verspannt das Düsenelement, welches endseitig gegen den Düsenträger anliegt, mit einem Düsenstock gegenüber einem Düsengrundköper unter Zwischenlage eines Dichtringes, der zwischen dem Grundkörper und einem Zwischenelement vorgesehen ist. In axialer Erstreckung befinden sich dementsprechend zwischen dem Düsengrundkörper und der Düsenhaube das Düsenelement, der Düsenstock und das Zwischenelement. Diese Bauteile sind lediglich in Längsrichtung des Düsenkanals gegeneinander verpresst.

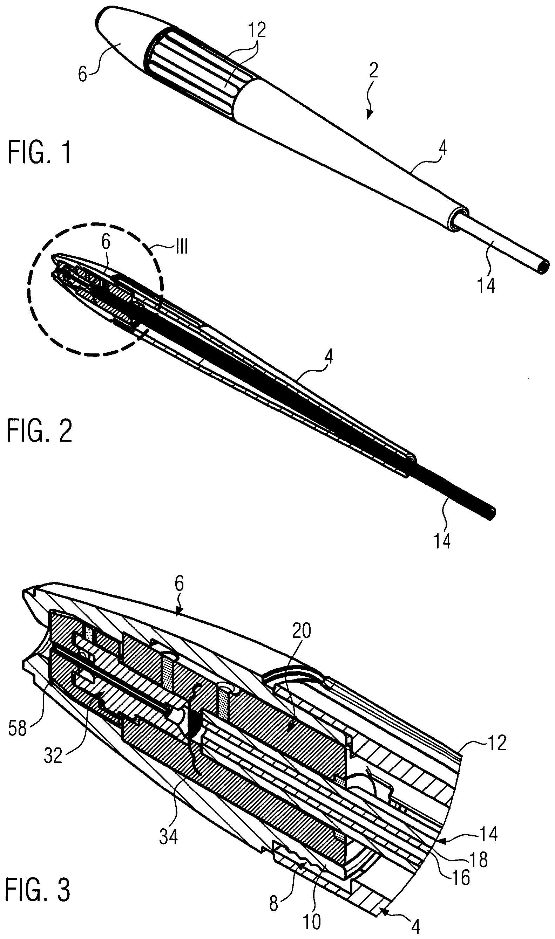

Die US 2,395,479 A offenbart eine Hülse, in welche eine Umhüllung aus Metall eingeschraubt ist, die nach dem Ausführungsbeispiel gemäß den Figuren 2 und 3 eine rohrförmige Düse in sich aufnimmt. Die Düse und die Umhüllung sind durch Zement miteinander verklebt bzw. verbunden.

Die US 2015/0209936 A1 betrifft ein Düsenelement, welches auf der Eingangsseite mit einer keramischen Beschichtung versehen und in einer Ausnehmung eines Düsenträgers aufgenommen ist. Das Düsenelement und der Düsenträger sind formschlüssig miteinander verbunden. Die Absätze 43 und 44 der D4 offenbaren, dass der Düsenträger aus einem gesinterten Metall hergestellt ist, wohingegen das Düsenelement aus einer Keramik ist.

Die US 3,705,693 A offenbart ein Düsenelement, welches in einem Düsenträger gehalten ist. Eine Dichtung liegt lediglich stirnseitig gegen den Düsenträger an, sodass dort eine fluiddichte Verbindung zwischen dem Düsenelement und dem Düsenträger gegeben ist. Eine Presspassung ist zwischen dem Düsenelement und dem Düsenträger vorgesehen.

Die US 1,393,562 A offenbart einen Düsenkörper aus gegossenem Metall, in welchen eine keramische Auskleidung eingegossen ist; Nach dem Ausführungsbeispiel ist die innere Auskleidung aus dem Düsenelement entfernbar. Beide Teile sind über eine Schraubverbindung miteinander verbunden.

Des Weiteren will die vorliegende Erfindung ein Einsetzteil für ein Handstück angeben, welches sich wirtschaftlich herstellen lässt. Das Einsetzteil ist dabei eine Untereinheit innerhalb des Handstücks und kann gegebenenfalls als Verbrauchsteil in einem als Mehrwegteil ausgebildeten Handstück zum Einsatz kommen.

Zur Lösung des Problems wird mit der vorliegenden Erfindung ein Handstück mit den Merkmalen von Anspruch 1 vorgeschlagen. Bei dem erfindungsgemäßen Handstück ist das Düsenelement fest mit einem einteiligen Düsenträger verbunden. Durch diese Verbindung ist es möglich, das relativ kleine Düsenelement sicher bei der Montage zu handhaben. Das Düsenelement des erfindungsgemäßen Handstücks hat eine Länge von üblicherweise nicht mehr als 30 mm, bevorzugt von zwischen 5 und 25 mm und einen Außendurchmesser von üblicherweise nicht mehr als 1 mm. Der Innendurchmesser des üblicherweise mit rundem Querschnitt vorgesehenen Strömungsdurchgangs des Düsenelementes kann zwischen 0,15 und 0,6 mm, bevorzugt zwischen 0,25 und 0,35 mm, besonders bevorzugt bei 0,3 ± 0,05 mm liegen.

Der Düsenträger ist aus Kunststoff ausgebildet und lässt sich dementsprechend leicht als Massenerzeugnis herstellen. Der Düsenträger ist des Weiteren dichtend an die Außenumfangsfläche des Düsenelementes angelegt, so dass der Düsenträger nicht nur eine verbesserte Handhabung im Rahmen der Montage erlaubt, sondern auch gleichzeitig die Abdichtung an der Außenumfangsfläche des Düsenelementes bewirkt, so dass auf gesonderte Dichtungsmittel verzichtet werden kann, um die Außenumfangsfläche bzw. zumindest einer der Stirnseite des Düsenelementes gegenüber dem strömenden Fluid abzudichten. Das Fluid wird üblicherweise an der Auslassseite des Düsenelementes an die Umgebung abgegeben. An dem gegenüberliegenden Einlassende liegt der wirkende Flüssigkeitsdruck an, der aufgrund der außenumfänglichen Abdichtung des Düsenelementes durch den Düsenträger stirnseitig gegen das Düsenelement anliegen kann, ohne dass zu besorgen ist, dass das Fluid an anderen Stellen entweicht als durch den innerhalb des Düsenelementes ausgebildeten Strömungskanal.

Dabei überragt üblicherweise das Auslassende des Düsenelementes den Düsenträger, wohingegen das Einlassende des Düsenelementes innerhalb des Düsenträgers aufgenommen ist, wodurch durch den Düsenträger der Einlass zu dem Düsenelement gebildet und abgedichtet wird, also speziell die den Einlass umgebenden Wandungen.

Gemäß der vorliegenden Erfindung ist der Düsenträger gegenüber dem Handstück festgelegt. Diese Festlegung kann unmittelbar durch Wechselwirkung zwischen dem Handstück und dem Düsenträger oder mittelbar durch weitere Teile eines vorgefertigten Einsatzteiles erfolgen, dessen Kernstück der Düsenträger mit dem Düsenelement ist, wobei sämtliche Elemente des Einsetzteiles üblicherweise fest miteinander verbunden, bevorzugt miteinander verklebt sind. Das Handstück bildet bei einer solchen Ausgestaltung üblicherweise lediglich ein Gehäuse, welches die den Fluidstrahl ausbildenden Komponenten umgibt und in für den Anwendungszweck geeigneter Weise ein Gehäuse bildet, das den ästhetischen, hygienischen und auch praktikablen Anforderungen genügt und dabei insbesondere von dem Benutzer des Handstücks sicher gehalten und geführt werden kann. Das Handstückgehäuse kann dabei ein- oder mehrteilig ausgebildet sein. Es ist üblicherweise als längliches Handstückgehäuse ausgeformt und weist an seinem auslassseitigen Ende eine Öffnung auf, die den Austritt des Fluidstrahls erlaubt, wohingegen das gegenüberliegende Ende üblicherweise eine Schlaucheinlassöffnung ausbildet, die den Schlauch mit wenig Spiel in das Innere des Handstückgehäuses überführt. Mit Blick auf die hohen Drücke ist der Schlauch dabei üblicherweise ein coexdrudierter Schlauch, d.h. ein Multilayer-Schlauch, bei dem in inneres Schlauchsegment aus einem Kunststoff mit hoher Festigkeit, beispielsweise Polyamid bestehen kann, wohingegen ein äußeres Schlauchsegment aus Polyurethan gebildet sein kann.

An der dem Handstück abgewandten Seite hat der Schlauch üblicherweise ein Kupplungselement, über welches das Handstück mit dem Schlauch an eine Pumpe anschließbar ist. Das Kupplungselement kann beispielsweise ein Luer-Kupplungselement sein.

Der Düsenträger kann kraft- und/oder formschlüssig mit dem Düsenelement verbunden sein. Der Düsenträger ist erfindungsgemäß einteilig ausgebildet, d.h. als einheitliches Bauteil mittels Kunststoff-Spritzgießen hergestellt. Das Düsenelement kann an seiner Außenumfangsfläche ausgebildete Vorsprünge aufweisen, um sicher zu verhindern, dass das Düsenelement aufgrund des einlassseitig wirkenden Flüssigkeitsdrucks aus dem Düsenträger herausgedrückt wird. Hierzu kann beispielsweise an dem Düsenelement ein radial nach außen vorspringender Kragen ausgebildet sein, der mit einer an dem Düsenträger ausgebildeten ringförmigen Gegenfläche zusammenwirkt, um die axiale Sicherung des Düsenelementes zu bewirken. Solche sich im Wesentlichen radial erstreckende Flächen können in besonderer Weise die Abdichtung zwischen dem Düsenelement und dem Düsenträger bewirken.

Mit Blick auf eine möglichst kostengünstige und einfache Herstellung ist der Düsenträger erfindungsgemäß durch Umspritzen des Düsenelementes mit diesem verbunden. Die Verbindung zwischen dem Düsenträger und dem Düsenelement ist dabei zumindest eine kraftschlüssige bedingt durch den gegen die Außenumfangsfläche des Düsenelementes, in der Regel unter Nachdruck aushärtenden Kunststoff.

Zumindest das Einsetzteil, bevorzugt das gesamte Handstück sind als Verbrauchsteile gedacht und so ist es für die vorliegende Erfindung bedeutsam, das Handstück insgesamt kostengünstig herzustellen. So wird üblicherweise ein Düsenelement verwendet, welches aus einem metallischen Rohr und einer bzw. mehreren endseitig dagegen verschweißten Blendenplatte besteht. Das Rohr ist dabei als Halbzeug vorbereitet und mit einem Kanal versehen, dessen Strömungsdurchmesser zwischen 0,15 und 0,6 mm liegt. Der Außendurchmesser des Rohres beträgt dabei üblicherweise nicht mehr als 1 mm. Ein solches Rohr lässt sich wirtschaftlich herstellen. Die Blendenplatte bildet bevorzugt einen Düsenkanal mit einem Innendurchmesser von zwischen 0,03 und 0,2 mm aus. Mit Blick auf die bevorzugte formschlüssige Verbindung zwischen dem Düsenträger und dem Düsenelement weist das Düsenelement an seiner Außenumfangsfläche eine Oberflächenkonturierung auf. Diese Oberflächenkonturierung kann beispielsweise durch Aufrauen der zunächst glatten metallischen Oberfläche des Düsenelementes erreicht werden. Das Aufrauen kann durch eine Strahlbehandlung, beispielsweise Sandstrahlen erfolgen. Bevorzugt wird indes eine Oberflächenkonturierung, die gezielt durch Lasergravieren aufgebracht wird. Mit Blick auf die gewünschte axiale Festlegung des Düsenelementes versteht sich, dass eine Oberflächenkonturierung insbesondere in Umfangsrichtung verlaufende Konturen aufweisen sollte. So kann über eine Lasergravierung an der Außenumfangsfläche eine in Umfangsrichtung umlaufende Nut oder eine ähnliche umfängliche Konturierung in die zunächst glatte Außenumfangsfläche des Düsenelementes eingebracht werden. Im Hinblick auf eine kostengünstige Fertigung bei gleichzeitiger guter Abdichtung und Verbindung zwischen dem Düsenelement und dem Düsenträger hat es sich als vorteilhaft erwiesen, zwischen ein und vier solcher umlaufender Konturierungen mit axialem Abstand verteilt an der Außenumfangsfläche des Düsenelementes vorzusehen.

Insbesondere beim Umspritzen des Düsenelementes mit flüssigem Kunststoff ergibt sich durch jegliche Oberflächenkonturierung eine verbesserte Verbindung und Abdichtung, da der schmelzflüssig eingebrachte Kunststoff in die radialen Vorsprünge bzw. Ausnehmungen an der Außenumfangsfläche des Düsenelementes eindringt und dort aushärtet.

Gemäß einer bevorzugten Weiterbildung der vorliegenden Erfindung ist dem Einlassende des Düsenelementes ein Strömungskanal vorgelagert, dessen Durchmesser größer als der Durchmesser des Düsenelementes ist und der in dem Düsenträger ausgebildet ist. Dieser Strömungskanal verjüngt sich üblicherweise in Strömungsrichtung und befindet sich regelmäßig zwischen dem Düsenelement und dem Schlauch zum Zuführen des aufzustrahlenden Fluids. Der in dem Düsenträger ausgebildete Strömungskanal kann dabei unmittelbar im Anschluss an das Düsenelement zylindrisch ausgebildet sein und sich beim Spritzgießen des Düsenträgers als Abformen eines Kerns ergeben, der stirnseitig gegen das Düsenelement anliegt, dieses abdichtet und für das Spritzgießen in Position hält. So befindet sich das Einlassende des Düsenelementes innerhalb des Düsenträgers. Das Auslassende des Düsenelementes überragt diesen mit einer Länge entsprechend zumindest dem dreifachen Durchmesser des Düsenelementes, bevorzugt dem fünf- bis zehnfachen Durchmesser des Düsenelementes, um insbesondere beim Umspritzen des Düsenelementes mit Kunststoff sicherzustellen, dass kein aus der den Düsenträger abformenden Kavität entlang der Außenumfangsfläche des Düsenelementes austretender Kunststoff das Auslassende des Düsenträgers verlegt. Ein den Düsenträger überragender Längenabschnitt des Düsenelementes dient beim Umspritzen auch der Fixierung und Positionierung des Düsenelementes mit dem Ziel, dieses nach Erstarren des den Düsenträger ausbildenden Kunststoffs auf der Mittellängsachse des Düsenträgers anzuordnen, so dass das Düsenelement nach dem Fügen sämtlicher Bauteile des Handstücks koaxial zu dem Schlauch angeordnet ist.

Zum Schutz des in dieser Weise den Düsenträger überragenden Düsenelementes wird gemäß einer bevorzugten Weiterbildung der vorliegenden Erfindung eine Düsenkappe vorgeschlagen, die den Düsenträger auslassseitig übergreift. Die Düsenkappe ist danach zumindest in Strömungsrichtung des zu fördernden Fluids hinter und damit auslassseitig des Düsenträgers vorgesehen. Die Düsenkappe hat üblicherweise eine Bohrung, die das Düsenelement nur teilweise aufnimmt. So übergreift die Düsenkappe vorzugsweise auch das stirnseitige Ende des Düsenelementes. Besonders bevorzugt bildet die Düsenkappe einen radial nach innen über die Außenumfangsfläche des Düsenelementes ragenden Flanschbereich aus, der üblicherweise eine Ringfläche ausformt, gegen welche die Stirnseite des Düsenelementes anliegt. Durch diese Anlage ergibt sich nach dem Fügen der Düsenkappe mit dem Düsenträger eine weitere formschlüssige Anlage des Düsenelementes, die das Düsenelement auch dann hält, wenn die Verbindung zwischen dem Düsenträger und dem Düsenelement aufgrund des wirkenden Flüssigkeitsdrucks bei der Behandlung versagen sollte. So ist ein weiteres Sicherungsmittel vorgesehen, um zu verhindern, dass das Düsenelement bei der Behandlung aus dem Handstück herausgedrückt wird. Die stirnseitige Anlage des Flanschbereiches gegen die Stirnseite des Düsenelementes erfolgt dabei üblicherweise im Rahmen der Montage, wenn die Düsenkappe über das auslassseitige Ende des Düsenelementes geschoben und mit dem Düsenträger verbunden, insbesondere verklebt wird. Ein hierbei wirkende axialer Druck gegen die Düsenkappe stellt sicher, dass die durch die Düsenkappe gebildete Ringfläche in der Regel ohne Spiel gegen die Stirnseite des Düsenelementes anliegt.

Insbesondere mit Blick auf das Verkleben von Düsenträger und Düsenkappe ist es zu bevorzugen, zwischen dem Düsenträger und der Düsenkappe einen Freiraum auszubilden, der bevorzugt von dem Düsenelement durchsetzt ist und der in der Lage ist, eventuell überschießenden Kleber und/oder ein Dichtelement aufzunehmen. Für diese Klebeverbindung sind üblicherweise stirnseitig zwischen dem Düsenträger und der Düsenkappe und/oder zwischen einer durch den Düsenträger gebildeten Außenumfangsfläche und einer durch die Düsenkappe gebildeten Innenumfangsfläche Spaltmaße vorgesehen, in die der Kleber eindringen und durch die Kleber fließen kann, um beispielsweise bei einer volumetrischen Dosierung des flüssigen Klebers eine gute Verteilung des Klebers an den Fügeflächen von Düsenträger und Düsenkappe zu erlauben. Besagter Freiraum sammelt dabei überschießenden Kleber. Er kann auch einen O-Ring aufnehmen, der durch das Aufsetzen der Düsenkappe gegen den Düsenträger verpresst und daher dichtend daran angelegt wird. Der Freiraum ist dabei bevorzugt als Ringraum ausgebildet und außenumfänglich von einem ringförmigen Kranz begrenzt, der durch den Düsenträger ausgebildet wird. Dieser ringförmige Kranz liegt üblicherweise endseitig in einer in der Düsenkappe ausgesparten ringförmigen Nut, die innenumfänglich durch einen Zentrierkranz der Düsenkappe begrenzt ist, welcher mit wenig Spiel in den Freiraum eingreift. Durch diesen Düsenkranz kann der O-Ring innerhalb des Freiraumes verpresst werden.

Zum Einbringen des die Düsenkappe mit dem Düsenträger verbindenden Klebers weist die Düsenkappe bevorzugt auf Höhe des Freiraumes zumindest eine bis zu dem Düsenträger reichende Klebeeinbringöffnung auf.

Der Kleber ist üblicherweise ein Einkomponenten-Kleber, der UV-härtbar ist. Mit Blick darauf sind die Düsenkappe und der Düsenträger üblicherweise aus einem transparenten Werkstoff, insbesondere einem transparenten Kunststoff wie Polycarbonat gebildet, so dass nach dem Einbringen des Klebers UV-Strahlung von außen bis zu dem Kleber aufgestrahlt werden kann.

Ein in dieser Weise vorbereiteter und aus dem Düsenträger und der Düsenkappe gebildeter Düsenelementhalter, der bevorzugt lediglich die drei Bauteile Düsenkappe, Düsenträger und Düsenelement umfasst, wird bevorzugt durch Vermittlung eines Adapterelementes mit dem Schlauch verbunden. Das Adapterelement hat eine zur Aufnahme des Düsenträgers angepasst ausgebildete Düsenträgeraufnahme und eine zur Aufnahme des Schlauches angepasst ausgebildete Schlauchaufnahme. Diese Aufnahmen sind in der Regel konzentrisch zueinander vorgesehen und üblicherweise durch einen Strömungskanal beabstandet, der durch das Adapterelement ausgeformt wird und üblicherweise absatzfrei in den durch den Düsenträger ausgebildeten Strömungskanal übergeht. Zwischen dem Düsenträger und dem Schlauch, bevorzugt zwischen dem Düsenträger und einer ringförmigen Anlagefläche des Adapterelementes befindet sich bevorzugt ein Filterelement, welches dafür Sorge trägt, das eventuell von der Behandlungsflüssigkeit mitgeschleppte Fremdkörper zurückgehalten werden und nicht bis zu dem Düsenelement gelangen, um eine Verstopfung des Düsenelementes zu vermeiden und/oder zu verhindern, dass entsprechende Fremdkörper in die zu behandelnde Wunde eingetragen werden. Ein solches Filterelement kann beispielsweise durch Umspritzen mittels Kunststoff mit dem Adapterelement verbunden oder als Einlegeteil daran vorgesehen sein.

Auch das Adapterelement ist bevorzugt aus Kunststoff, speziell einem lichtdurchlässigen Kunststoff gebildet. Die Anordnung des Filterelementes an einer Ringfläche des Adapterelementes bietet die Möglichkeit, das Filterelement zwischen zwei Stempeln zu fixieren, die als bewegliche Kerne in die das Adapterelement ausformende Kavität eingebracht werden, bevor der das Adapterelement ausformende Kunststoff eingespritzt wird. Zwischen diesen Stempeln wird das Filterelement geklemmt und ist nach dem Erstarren des Kunststoffs in dem Adapterelement auf einfache Weise unverlierbar aufgenommen. Dabei bildet der auslassseitig gegen das Filterelement anliegende Stempel regelmäßig die Düsenträgeraufnahme aus, wohingegen der von der Einlassseite einwirkende Stempel die Schlauchaufnahme, regelmäßig auch den gegenüber dem Durchmesser der Schlauchaufnahme im Durchmesser verringerten und innerhalb des Adapterelementes ausgebildeten Strömungskanal ausformt.

Der Düsenträger und/oder der Schlauch sind bevorzugt mit dem Adapterelement verklebt. Dazu weist das Adapterelement bevorzugt eine zu dem Düsenträger reichende Klebeeinbringöffnung und/oder eine bis zu dem Schlauch reichende Klebeeinbringöffnung auf. Bevorzugt erstrecken sich sämtliche Einbringöffnungen für Kleber gemäß Weiterbildungen der vorliegenden Erfindung in radialer Richtung. Der Kleber wird grundsätzlich volumetrisch zudosiert, so dass sichergestellt wird, dass eine vorbestimmte Menge an Kleber eingespritzt wird, um die Teile zu fügen. Um zu verhindern, dass ein radial eingespritzter und das Adapterelement mit dem Düsenträger verbindender Kleber stirnseitig den Strömungsweg zwischen dem Düsenträger und dem Adapter verlegt und/oder in das Filterelement eindringt, wird das Filterelement üblicherweise eine Ringfläche des Adapterelementes überdeckend vorgesehen, gegen welche der Düsenträger angedrückt wird bzw. anliegt. Eventuell von der Außenumfangsfläche des Düsenträgers in diesen Bereich vordringender Kleber wird dementsprechend am radial äußeren Bereich dieser Ringfläche aufgehalten, da das relativ weiche Filtermaterial des Filterelementes wie ein Dichtelement wirkt, welches zwischen dem Adapterelement und dem Düsenträger eingeklemmt ist. Auch kann das Adapterelement mit dem Düsenträger bzw. dem Schlauch durch Umspritzen von Düsenträger bzw. Adapterelement und/oder Schlauch mit einem das Adapterelement oder den Düsenträger ausbildenden Kunststoff verbunden sein.

Die Schlauchaufnahme endet ebenfalls bevorzugt an einer durch das Adapterelement gebildeten Ringfläche. Diese Ringfläche bildet einen Anschlag für den in das Adapterelement eingeführten Schlauch aus. Die die Ringfläche begrenzende und durch das Adapterelement ausgebildete Innenumfangsfläche ist leicht konisch zum Strömungsauslass hin verjüngt, so dass der in die Schlauchaufnahme eingebrachte Schlauch radial leicht verpresst wird. Durch diese Ausgestaltung ergibt sich eine Abdichtung, die sicher verhindert, dass der radial auf Höhe des Schlauches eingebrachte Kleber an die Stirnseite des Schlauches gelangt und dort den Strömungsweg beeinträchtigen kann.

Durch angepasste Ausbildung des in dem Adapterelement ausgebildeten Strömungskanals und/oder des in dem Düsenträger ausgebildeten Strömungskanals ist dieser in Richtung auf das Einlassende des Düsenelementes hin verjüngt bevorzugt. Bevorzugt hat dabei der in dem Düsenträger ausgebildete Strömungskanal einen konischen Abschnitt, der den größeren Außendurchmesser des in dem Adapterelement ausgebildeten Strömungskanals in den insofern verkleinerten Außendurchmesser des in dem Düsenträger unmittelbar in Strömungsrichtung vor dem Düsenelement ausgebildeten Strömungskanals überführt.

Bevorzugt sind die eine bestimmte Form des Fluidstrahls vorgebenden Teile des Handstücks in vorbestimmter Weise relativ zueinander radial positioniert, so dass ein das Handstück handhabender Benutzer bei einer bestimmten radialen Ausrichtung des Handstücks sicher davon ausgehen kann, dass diese radiale Ausrichtung beispielsweise die Ausrichtung eines flachen Düsenstrahls in vorbestimmter Weise vorgibt. Hierzu hat das Adapterelement bevorzugt an seiner Außenumfangsfläche ein Formschlusselement, welches mit einem an dem Handstück und/oder dem Düsenträger vorgesehenen Formschlussgegenelement zur radialen Positionierung des Adapterkörpers zusammenwirkt. Formschlusselement und Formschlussgegenelement sind bevorzugt durch an der Innenumfangs- bzw. Außenumfangsfläche sich in radialer Richtung erstreckende Stege gebildet, die in korrespondierend hierzu ausgebildete Ausnehmungen eingreifen.

Das mit dem nebengeordneten Aspekt der vorliegenden Erfindung unter Schutz gestellte Einsetzteil für ein Handstück ist in Anspruch 13 definiert. Dieses Einsetzteil kann als Verbrauchsteil kostengünstig hergestellt sein, insbesondere sofern die zuvor mit Rücksicht auf den Düsenträger und die Düsenkappe diskutierten Weiterbildungen verwirklicht sind. Ein in solcher Weise hergestelltes Einsetzteil wird über das Adapterelement mit dem Schlauch verbunden und kann für sich das Handstück und damit das Handstückgehäuse ausbilden oder aber in ein separates, an ergonomische Anforderungen angepasstes Handstückgehäuse eingesetzt sein.

Mit der vorliegenden Offenbarung wird ferner ein Verfahren zum Herstellen eines Handstücks zum Auftragen eines Fluidstrahls angegeben. Bei diesem Vorgehen wird zunächst das Düsenelement als separates Bauteil vorbereitet. Dazu wird regelmäßig ein Rohr mit zumindest einer Blendenplatte verbunden. Die Blendenplatte ist aus einem dünnen Blech geschnitten, bevorzugt mittels Laserschneiden und mit einer die Strahlgeometrie vorgebenden Blendenöffnung, gegebenenfalls auch mit mehreren Blendenöffnungen versehen. Dabei wird beim Schneiden der Blendenplatte aus einem größeren Blechstück diese zunächst nicht vollständig von der Blechplatte entfernt. Vielmehr verbleiben Radialstege, um die Blendenplatte mit dem Blechstück verbunden zu halten. Beim Laserschweißen der Blendenplatte gegen das metallische Rohr werden diese Stege durchtrennt, so dass die Blendenplatte danach allein dem Rohr zugeordnet und mit diesem verbunden ist. Das Düsenelement wird mit einem einteiligen Düsenträger so verbunden, dass der Düsenträger dichtend an der Außenumfangsfläche des Düsenelementes anliegt. Dabei wird der Düsenträger besonders bevorzugt mittels Umspritzen dichtend mit dem Düsenelement verbunden. Die so hergestellte handhabbare Untereinheit des Handstücks wird danach in der zuvor beschriebenen Weise mit einer Düsenkappe verbunden, bevorzugt verklebt, bevorzugt unter Zwischenlage eines O-Rings, der zwischen der Düsenkappe und dem Düsenträger angeordnet und dazwischen verspannt wird und von dem Düsenelement bevorzugt durchsetzt ist. Der Düsenträger und die Düsenkappe werden bevorzugt miteinander verklebt, um einen für sich zu handhabenden Düsenelementhalter auszubilden. Der Düsenelementhalter vereinigt dabei das Düsenelement, die Düsenkappe und den Düsenträger in sich, wobei die Düsenkappe das auslassseitige Ende des Düsenelementes umgibt und schützt. In dieser Weise vorbereitet wird der Düsenelementhalter in ein bevorzugt mittels Spritzgießen ausgebildetes Adapterelement eingesetzt. Auch wird das auslassseitige Ende eines Schlauches in das Adapterelement eingesetzt. Danach wird bevorzugt durch volumetrisches Zudosieren von Kleber das Adapterelement mit dem Düsenelementhalter einerseits und dem Schlauch andererseits verklebt. Der Kleber wird bevorzugt mittels UV-Strahlen ausgehärtet, wozu der Düsenelementhalter und/oder das Adapterelement bevorzugt aus UV-durchlässigem Kunststoff gebildet sind.

Weitere Einzelheiten und Vorteile der vorliegenden Erfindung ergeben sich aus der nachfolgenden Beschreibung eines Ausführungsbeispiels in Verbindung mit der Zeichnung. In dieser zeigen:
- Figur 1: eine perspektivische Seitenansicht eines Ausführungsbeispiels des vorderen Bereichs eines Handstücks;
- Figur 2: eine Längsschnittansicht des in Figur 1 gezeigten Ausführungsbeispiels in perspektivischer Darstellung;
- Figur 3: das in Figur 2 vergrößerte Detail III;
- Figur 4: das in Figur 3 gezeigt Detail in einer nicht perspektivischen Längsschnittansicht;
- Figur 5: das Detail V gemäß Figur 4;
- Figur 6: das Detail VI gemäß Figur 4;
- Figur 7: eine Schnittdarstellung entlang der Linie VII-VII gemäß Figur 4;
- Figur 8: eine Schnittdarstellung entlang der Linie VIII-VIII gemäß Figur 4;
- Figur 9: eine Schnittdarstellung entlang der Linie IX-IX gemäß Figur 4 und
- Figur 10: eine gegenüber dem vorbeschriebenen ersten Ausführungsbeispiel leicht veränderte Variante als zweites Ausführungsbeispiel.

In der Zeichnung kennzeichnet Bezugszeichen 2 ein Handstückgehäuse mit einem Griffstiel 4 und einer Gehäusekappe 6, die über ein Figur 3 mit Bezugszeichen 8 gekennzeichnete Formschlussverbindung verrastet sind, wozu die Gehäusekappe 6 einen in den Griffstiel 4 eingreifenden Befestigungszapfen 10 aufweist, der an seiner Außenumfangsfläche mit Rippen versehen ist, die mit einer korrespondierend hierzu ausgebildeten Gestaltung an der Innenumfangsfläche des Griffstiels 4 zusammenwirken.

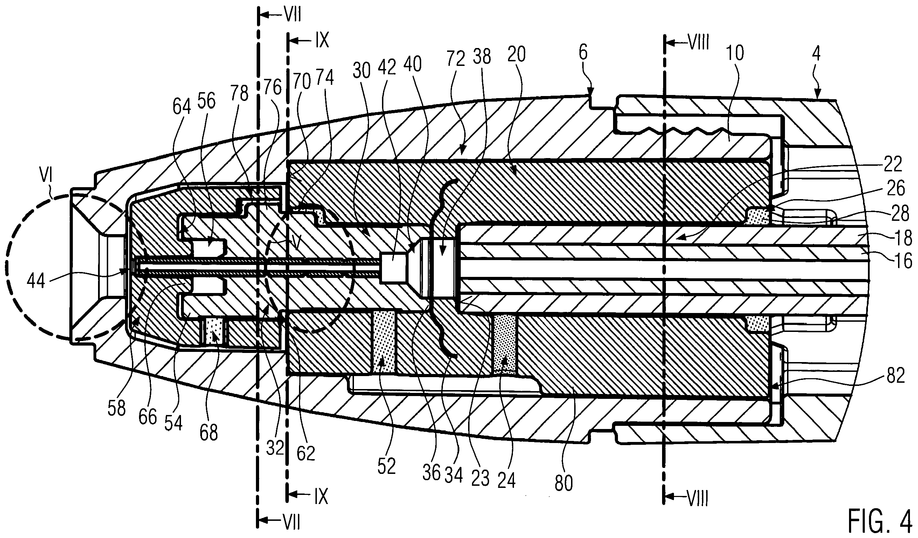

Der Griffstiel 4 hat an seinem vorderen, auslassseitigen Ende sich axial erstreckende und an der Außenumfangsfläche vorgesehene Rippen 12, die auf dem Umfang verteilt vorgesehen sind und die die haptischen Eigenschaften des Griffstiels 4 verbessern. Ein das Handstückgehäuse 2 haltender Benutzer umgreift dort das Handstückgehäuse 2 zwischen seinen Fingern. Das einlassseitige hintere Ende des Handstückgehäuses 2 liegt dabei in der Hand des Benutzers und wird von einem Schlauch 14 überragt, der als coextrudierter Schlauch mit einem inneren Schlauchabschnitt 16 aus Polyamid und einem äußeren Schlauchabschnitt 18 aus Polyurethan versehen ist (vgl. Figur 3, 4).

Mit seinem auslassseitigen Ende ist der Schlauch 14 in einem Adapterelement 20 aufgenommen, welches hierzu eine zur Aufnahme des Schlauches angepasst ausgebildete Schlauchaufnahme 22 hat. Die Schlauchaufnahme 22 ist im Wesentlichen zylindrisch und an den Außenumfang des Schlauches 14 so angepasst, dass ein einem Spalt zwischen dem Schlauch 14 und der Schlauchaufnahme 22 Kleber eindringen kann. Das strömungsauslassseitige Ende der Schlauchaufnahme 22 ist konisch radial nach innen verjüngt, so dass der Schlauch 14 an seinem auslassseitigen Ende radial verpresst wird. Mit axialem Abstand zu einer durch das Adapterelement 20 gebildeten Ringschulter 23 weist das Adapterelement 20 eine zu dem Schlauch 14 reichende Klebeeinbringöffnung 24 auf, die als Radialbohrung in dem Adapterelement 20 versehen ist. Durch das konisch zulaufende Ende der Schlauchaufnahme 22 kann ein hierdurch eingebrachter Kleber lediglich axial bis zu dem auslassseitigen Ende des Schlauches 14 gelangen, nicht aber an dessen Stirnseite. Der eingebrachte Kleber kann in axialer Richtung den gesamten radialen Spalt zwischen dem Schlauch 14 und der Schlauchaufnahme 22 ausfüllen. Zur Überprüfung einer hinreichenden Verklebung ist an der freien Stirnseite des Adapterelementes 20 ein den Schlauch 14 umgebender Ringraum 26 ausgespart, der beim Verkleben mit Kleber 28 ausgefüllt wird. Das Ansammeln des Klebers 28 in diesem Ringraum 26 kennzeichnet eine hinreichende Ausfüllung des Ringspaltes mit Kleber und damit eine zuverlässige Verklebung des Schlauches 14 gegenüber dem Adapterelement 20.

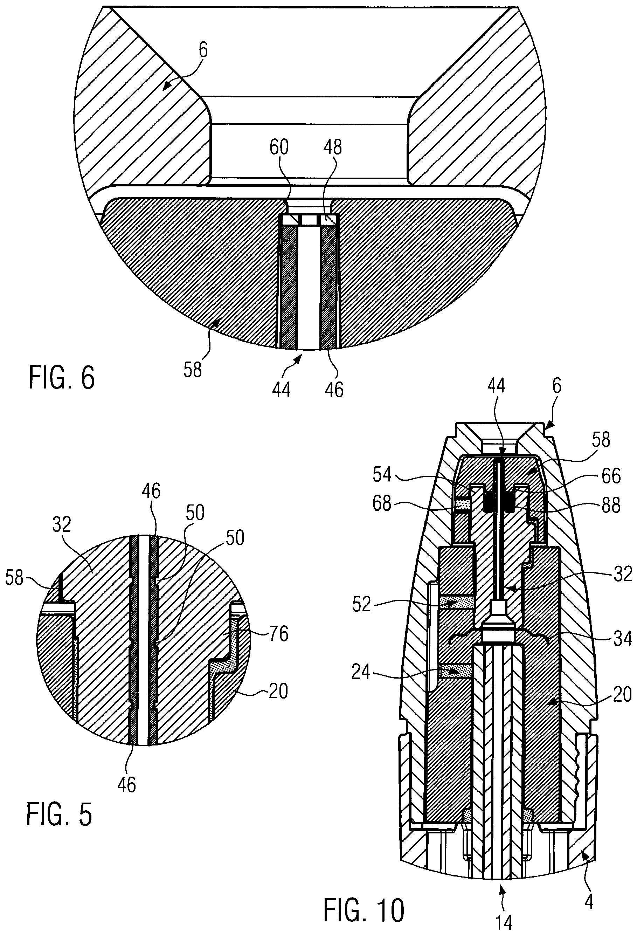

An dem gegenüberliegenden Ende bildet das Adapterelement 20 eine Düsenträgeraufnahme 30 aus, die zur Aufnahme eines mit Bezugszeichen 32 gekennzeichneten Düsenträgers ausgebildet ist. Dieser Düsenträger 32 liegt unter Zwischenlage eines Filterelementes 34 gegen eine weitere, durch das Adapterelement 20 ausgebildete Ringschulter 36 an. Stromaufwärts des Filterelementes 34 formt das Adapterelement 20 einen zylindrischen Strömungskanal 38 aus, der mit dem Filterelement 34 endet und in einen in den Düsenträger 32 ausgesparten Strömungskanal 40 übergeht, der einen Konusabschnitt aufweist, durch welchen der Durchmesser des Strömungskanals 38 in einen Durchmesser eines zylindrischen Kanalabschnitts 42 des Strömungskanals 40 überführt wird, der in Strömungsrichtung unmittelbar einem Düsenelement 44 vorgelagert vorgesehen ist. Das Düsenelement 44 besteht aus einem Rohrkörper 46, an dessen Ende eine Blendenplatte 48 angeschweißt ist (vgl. Figur 6). Der Rohrkörper 46 und die Blendenplatte 48 bestehen aus Metall.

Wie die Figuren 3 und 4 verdeutlichen, ist das Einlassende des Düsenelementes 44 innerhalb des Düsenträgers 32 vorgesehen. Der zylindrische Kanalabschnitt 42 hat einen größeren Durchmesser als der Außendurchmesser des Düsenelementes 44, so dass das geförderte Fluid mit seinem Druck stirnseitig gegen das Düsenelement 44 anliegt. Das Düsenelement 44 ist fest und fluiddicht mit dem Düsenträger 32 verbunden, vorliegend durch Umspritzen des Rohrkörpers 46, der hierzu an seiner Außenumfangsfläche eine Oberflächenkonturierung aufweist, die in Figur 5 verdeutlicht ist. Der Rohrkörper 46 hat mehrere in Umfangsrichtung umlaufend an der Außenumfangsfläche durch Lasergravieren ausgesparte Nuten 50, in welchen der schmelzflüssig eingebrachte und den Düsenträger 32 ausbildende Kunststoff erstarrt, um zwischen dem Düsenelement 44 und dem Düsenträger 32 eine formschlüssige Verbindung herzustellen. Die Verbindung zwischen dem Düsenträger 32 und dem Düsenelement 44 ist auch fluiddicht. So wird das mit dem Systemdruck in dem zylindrischen Kanalabschnitt 42 anstehende Fluid allein durch das Düsenelement 44 herausgelassen und durch Formung in der Blendenplatte 48 mit einer vorbestimmten Strahlgeometrie auf eine zu behandelnde Wunde aufgestrahlt.

Das Adapterelement 20 hat eine zweite Klebeeinbringöffnung 52, die als radiale Bohrung in dem Adapterelement 20 ausgespart ist und mit axialem Abstand zu der Ringschulter 36 vorgesehen ist. Dieser axiale Abstand entspricht in etwa dem axialen Abstand der Klebeeinbringöffnung 24 zu der Ringschulter 23.

An seinem auslassseitigen Ende hat der Düsenträger 32 einen ringförmigen Kranz 54, der das Düsenelement 44 umfänglich mit Abstand zur Ausbildung eines Freiraumes 56 umgibt. Dieser Freiraum 56 ist als Ringraum ausgeformt und wird außenumfänglich durch den ringförmigen Kranz 54 und innenumfänglich durch die Außenumfangsfläche des Düsenelementes 44 begrenzt.

Das auslassseitige Ende des Düsenelementes 44 ist in einer Düsenkape 58 aufenommen, die - wie Figur 6 verdeutlicht - einen Flanschbereich 60 ausformt, der das Düsenelement 44 stirnseitig übergreift und mit einer durch den Flanschbereich 60 ausgebildeten Ringfläche stirnseitig gegen die Blendenplatte 48 anliegt und dementsprechend eine formschlüssige Sicherung des Düsenelementes 44 bereitstellt, die verhindert, dass das Düsenelement 44 in axialer Richtung aufgrund des wirkenden Flüssigkeitsdruckes aus dem Düsenträger 32 austreten kann.

Die Düsenkappe 58 bildet eine im Wesentlichen zylindrische Aufnahme 62 für das auslassseitige Ende des Düsenträgers 32 aus. Diese Aufnahme 62 umgibt den Düsenträger 32 radial. Die Düsenkappe 58 hat an dem stirnseitigen Ende dieser Aufnahme 62 eine ringförmige Nut 64, die zur Aufnahme des ringförmigen Kranzes 54 angepasst ausgebildet ist und radial innen durch einen Zentrierkranz 66 begrenzt ist, der in den ringförmigen Freiraum 56 vorspringt.

Die Düsenkappe 58 hat eine sich radial erstreckende Klebeeinbringöffnung 68, die in axialer Richtung in etwa auf Höhe des ringförmigen Freiraumes 56 vorgesehen ist. Diese Klebeeinbringöffnung hat wiederum einen axialen Abstand von dem auslassseitigen Ende des Düsenträgers 32 gebildet durch den ringförmigen Kranz 54 einerseits und einem einlassseitigen Ende der Düsenkappe 58 andererseits. Zwischen der Düsenkappe 58 und dem Düsenträger 32 ist ein geringes Spaltmaß vorgesehen, so dass der durch die Klebeeinbringöffnung 68 eingebrachte Kleber sich sowohl im Bereich der Aufnahme 62 zwischen der Innenumfangsfläche der Düsenkappe 58 und dem Düsenträger 62 wie auch stirnseitig zwischen dem Düsenträger 32 und der Düsenkappe 58 ausbreiten kann. Eventuell überschüssiger Kleber kann in den Freiraum 56 gelangen, ohne dass zu befürchten ist, dass dieser das Düsenelement 44 auslassseitig verlegt. Der Freiraum 56 ist dementsprechend als Reservoir zur Aufnahme überschüssigen Klebers ausgebildet.

Wie die Figuren 3 und 4 verdeutlichen, hat die Gehäusekappe 6 eine Anlageschulter 70, gegen welche das Adapterelement 20 stirnseitig anliegt, wodurch das Adapterelement 20 und damit der damit verklebte Düsenträger 32 mit der damit verklebten Düsenkappe 58 axial fixiert ist.

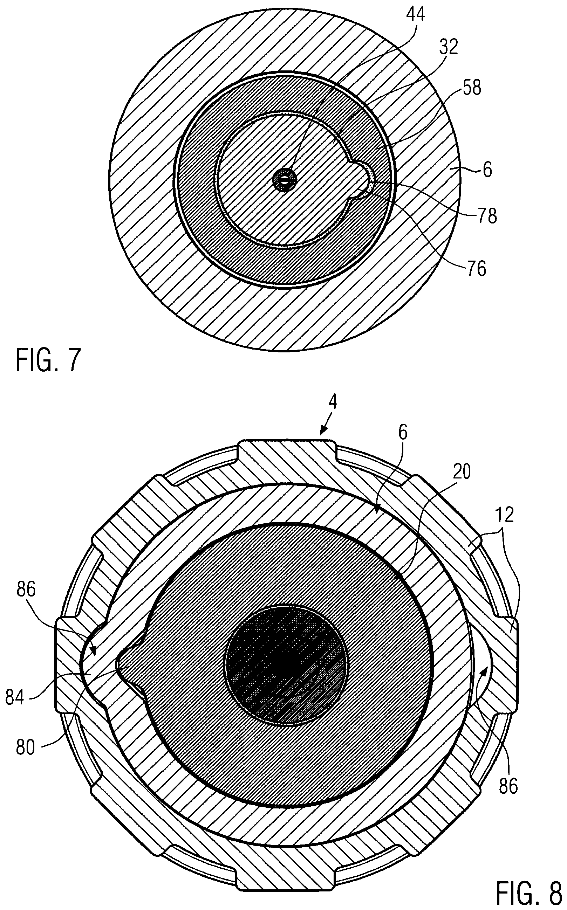

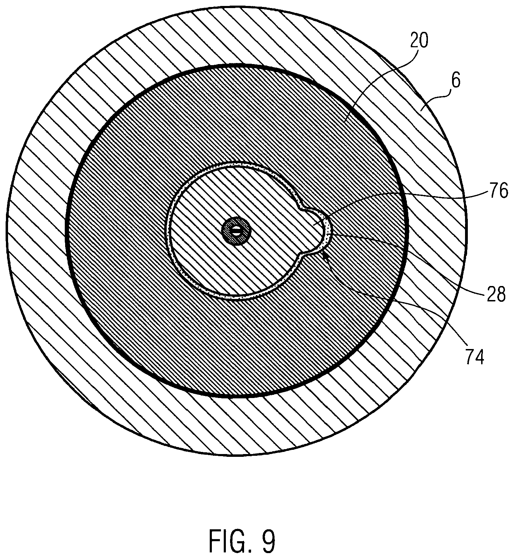

Bei der Herstellung des Ausführungsbeispiels wird zunächst das transparente und den Düsenträger 32 ausbildende Kunststoffmaterial in eine Spritzgießform eingespritzt, in welche das Düsenelement 44 einlassseitig hineinragt. Ein den Strömungskanal 40 ausbildender Stempel liegt dabei endseitig gegen das Düsenelement 44 dichtend an, so dass dort kein Kunststoff in das Düsenelement 44 eindringen kann. Die so erstellte Einheit aus Düsenträger 32 und Düsenelement 44 wird entformt. Danach wird die zunächst separat ebenfalls mittels Spritzgießen hergestellte Düsenkappe 58 über das auslassseitige Ende des Düsenelementes 44 geschoben. Durch die Klebeeinbringöffnung 68 wird UVaushärtbarer Einkomponenten-Kleber eingebracht, der sich zwischen dem Düsenträger 32 und der Düsenkappe 58 ausbreitet und durch UV-Bestrahlung ausgehärtet wird. Auf diese Weise ist eine separat handhabbare Untereinheit in Form eines Düsenelementhalters 72 geschaffen. Dieser Düsenelementhalter 72 hat Formschlussmittel zur radialen Positionierung des Düsenträgers 32 in dem Adapterelement 20, die in Figur 3 bzw. 4 im perspektivischen Längsschnitt bzw. im Längsschnitt und in Figur 7 und 9 im Querschnitt verdeutlicht sind. Das Adapterelement 20 hat an seiner auslassseitigen Öffnung, die zu der Düsenträgeraufnahme 30 führt, eine sich axial erstreckende Positioniernut 74, in die eine die im Wesentlichen zylindrische Außenumfangsfläche des Düsenträgers 32 überragende Positionierrippe 76 hineinragt (Figur 9). Die Positionierrippe 76 ist in axialer Richtung verlängert bis in die Düsenkappe 58 und dort im Eingriff in einer mit Bezugszeichen 78 in Figur 7 gekennzeichneten Positioniernut, die an der Düsenkappe 58 innenumfänglich ausgespart ist.

Durch diese Ausgestaltung ist die radiale Positionierung der Düsenkappe 58 relativ zu dem Düsenträger 32 und die radiale Positionierung des Düsenträgers 32 und damit des vormontierten Düsenelementhalters 72 relativ zu dem Adapterelement 20 vorgegeben.

Wie Figur 8 verdeutlicht, hat das Adapterelement 20 an seiner Außenumfangsfläche eine entsprechende Ausgestaltung zur radialen Positionierung in Form einer als Formschlusselement an dem Adapterelement 20 vorgesehenen Positionierrippe 80, die in eine Positioniernut 82 eingreift, die an der Gehäusekappe 6 ausgespart ist. An dieser Position ragt von der Außenumfangsfläche der Gehäusekappe 6 eine weitere Positionierrippe 84 hervor, die in einer weiteren Positioniernut 86 im Eingriff ist, die an der Innenumfangsfläche des Griffstiels 4 ausgespart ist. Die entsprechende Weiterbildung ermöglicht die genaue Positionierung eines mit einer in Figur 7 erkennbaren Breitschlitzdüse ausgebildeten Düsenelementes relativ zu dem Handstückgehäuse 2. Zur Vereinfachung der Montage hat der Griffstiel 4 zwei mit einem Winkel von 180° versetzt vorgesehene Positioniernuten 86, die wahlweise mit der Positionierrippe 84 zusammenwirken können, ohne die gewünschte relative Ausrichtung der Breitschlitzdüse zu einem Positionsindikator, der an dem Handstückgehäuse 2 vorgesehen sein kann, zu verfälschen.

Die Figur 10 zeigt eine Schnittansicht entsprechend Figur 4 für ein alternatives Ausführungsbeispiel. Gleiche Bauteile sind mit gleichen Bezugszeichen gekennzeichnet. Dieses alternative Ausführungsbeispiel unterscheidet sich von dem zuvor diskutierten Ausführungsbeispiel durch einen O-Ring 88, der in den Freiraum 56 eingesetzt ist und zwischen dem Zentrierkranz 66 und einem ringförmigen Bogen des Freiraumes 56 geklemmt ist, um einerseits abgedichtet gegen die Außenumfangsfläche des diesen O-Ring 88 durchragenden Düsenelementes 44 und andererseits gegen die Innenumfangsfläche des ringförmigen Kranzes 54 abgedichtet anzuliegen. Durch diesen O-Ring wird eine Sicherung geschaffen für den Fall, dass im Rahmen des Betriebs die umfängliche Abdichtung des Düsenelementes 44 über den Düsenträger 32 Schaden nimmt.

### Bezugszeichenliste

- 2: Handstückgehäuse
- 4: Griffstiel
- 6: Gehäusekappe
- 8: Formschlussverbindung
- 10: Befestigungszapfen
- 12: Rippe
- 14: Schlauch
- 16: innerer Schlauchabschnitt
- 18: äußerer Schlauchabschnitt
- 20: Adapterelement
- 22: Schlauchaufnahme
- 23: Ringschulter
- 24: Klebeeinbringöffnung
- 26: Ringraum
- 28: Kleber
- 30: Düsenträgeraufnahme
- 32: Düsenträger
- 34: Filterelement
- 36: Ringschulter
- 38: Strömungskanal
- 40: Strömungskanal
- 42: zylindrischer Kanalabschnitt des Strömungskanals 40
- 44: Düsenelement
- 46: Rohrkörper
- 48: Blendenplatte
- 50: Nut
- 52: Klebeeinbringöffnung
- 54: ringförmiger Kranz
- 56: Freiraum
- 58: Düsenkappe
- 60: Flanschbereich
- 62: Aufnahme
- 64: ringförmige Nut
- 66: Zentrierkranz
- 68: Klebeeinbringöffnung
- 70: Anlageschulter
- 72: Düsenelementhalter
- 74: Positioniernut des Adapterelementes 20
- 76: Positionierrippe des Düsenträgers 32
- 78: Positioniernut der Düsenkappe 58
- 80: Positionierrippe des Adapterelementes 20
- 82: Positioniernut der Gehäusekappe 6
- 84: Positionierrippe der Gehäusekappe 6
- 86: Positioniernut des Griffstiels 4
- 88: O-Ring

## Patentansprüche

1. Handstück zum Aufstrahlen eines Fluidstrahls mit einem Handstückgehäuse (2) in dem ein Düsenelement (44) zum Ausbilden der Strahlgeometrie des Fluidstrahls aufgenommen ist, wobei das Düsenelement (44) fest mit einem einteiligen Düsenträger (32) verbunden und gegenüber dem Handstückgehäuse (2) festgelegt ist, **dadurch gekennzeichnet, dass** der Düsenträger (32) aus Kunststoff gebildet und mittels Umspritzen mit dem Düsenelement (44) verbunden ist und dichtend an der Außenumfangsfläche des Düsenelementes (44) anliegt.

2. Handstück nach Anspruch 1, **dadurch gekennzeichnet, dass** das Düsenelement (44) mit seinem Auslassende den Düsenträger (32) überragt und mit seinem Einlassende innerhalb des Düsenträgers (32) aufgenommen ist.

3. Handstück nach einem der vorherigen Ansprüche, **dadurch gekennzeichnet, dass** das Düsenelement (44) an seiner Außenumfangsfläche eine Oberflächenkonturierung zur formschlüssigen Verbindung zwischen dem Düsenträger (32) und dem Düsenelement (44) aufweist.

4. Handstück nach einem der vorherigen Ansprüche, **dadurch gekennzeichnet, dass** einem Einlassende des Düsenelementes (44) ein in dem Düsenträger (32) ausgebildeter Strömungskanal (40) vorgelagert ist, dessen Durchmesser größer als der Außendurchmesser des Düsenelementes (44) ist.

5. Handstück nach einem der vorherigen Ansprüche, **gekennzeichnet durch** eine Düsenkappe (58), die den Düsenträger (32) auslassseitig übergreift.

6. Handstück nach Anspruch 5, **dadurch gekennzeichnet, dass** zwischen dem Düsenträger (32) und der Düsenkappe (58) ein von dem Düsenelement (44) durchsetzter Freiraum (56) ausgebildet ist.

7. Handstück nach einem der Ansprüche 5 oder 6, **gekennzeichnet durch** einen O-Ring (88), der zwischen der Düsenkappe (58) und dem Düsenträger (32) vorgesehen und von dem Düsenelement (44) durchsetzt ist.

8. Handstück nach einem der vorherigen Ansprüche, **gekennzeichnet durch** ein Adapterelement (20), das eine zur Aufnahme des Düsenträgers (32) angepasst ausgebildete Düsenträgeraufnahme (30) und eine zur Aufnahme eines mit dem Düsenelement (44) kommunizierenden Schlauches (14) angepasst ausgebildete Schlauchaufnahme (22) aufweist.

9. Handstück nach Anspruch 8, **dadurch gekennzeichnet, dass** der Düsenträger (32) und/oder der Schlauch (14) mit dem Adapterelement (20) verklebt und/oder durch Umspritzen verbunden ist.

10. Handstück nach Anspruch 9, **dadurch gekennzeichnet, dass** das Adapterelement (20) eine bis zu dem Düsenträger (32) reichende Klebeeinbringöffnung (52) und/oder eine bis zu dem Schlauch (14) reichende Klebeeinbringöffnung (24) aufweist.

11. Handstück nach einem der Ansprüche 8 bis 10, **dadurch gekennzeichnet, dass** zwischen dem Düsenträger (32) und dem Schlauch (14) ein Filterelement (34) angeordnet ist.

12. Handstück nach einem der Ansprüche 8 bis 11, **dadurch gekennzeichnet, dass** das Adapterelement (20) an seiner Außenumfangsfläche mit einem Formschlusselement (80) versehen ist, welches mit einem an dem Handstück (2) und/oder an dem Düsenträger (32) vorgesehenen Formschlussgegenelement (82) zur radialen Positionierung des Adapterkörpers (20) zusammenwirkt.

13. Einsetzteil für ein Handstück nach einem der vorherigen Ansprüche mit einem Düsenelement (44) und einem fest mit einer Außenumfangsfläche des Düsenelementes (44) verbundenen einteiligen Düsenträger (32), der aus Kunststoff gebildet und mittels Umspritzen mit dem Düsenelement (44) verbunden ist und abgedichtet an der Außenumfangsfläche des Düsenelements (44) anliegt.

14. Einsetzteil nach Anspruch 13, **gekennzeichnet durch** zumindest ein kennzeichnendes Merkmal der Ansprüche 2 und 3 bis 7.

## Claims

1. Handpiece for spraying on a fluid jet with a handpiece casing (2) in which a nozzle element (44) for forming the jet geometry of the fluid jet is accommodated wherein said nozzle element (44) is fixedly connected to an integrally formed nozzle holder (32) and is fixed relative to said handpiece casing (2), **characterized in that** said nozzle holder (32) is formed from plastic material and is connected to said nozzle element (44) by way of overmolding and sealingly abuts against the outer circumferential surface of said nozzle element (44).

2. Handpiece according to claim 1, **characterized in that** said nozzle element (44) projects with its outlet end over said nozzle holder (32) (and is received with its inlet end within said nozzle element (32)).

3. Handpiece according to one of the preceding claims, **characterized in that** said nozzle element (44) comprises a surface contour on its outer circumferential surface for a positive-fit connection between said nozzle holder (32) and said nozzle element (44).

4. Handpiece according to one of the preceding claims, **characterized in that** a flow channel (40), which is formed in said nozzle holder (32) and the diameter of which is greater than the outer diameter of said nozzle element (44), is disposed upstream of an inlet end of said nozzle element (44).

5. Handpiece according to one of the preceding claims, **characterized by** a nozzle cap (58) which reaches over said nozzle holder (32) on the outlet side.

6. Handpiece according to claim 5, **characterized in that** a free space (56) penetrated by said nozzle element (44) is formed between said nozzle holder (32) and said nozzle cap (58).

7. Handpiece according to one of the claims 5 or 6, **characterized by** an O-ring (88) which is provided between said nozzle cap (58) and sad nozzle holder (32) and penetrated by said nozzle element (44).

8. Handpiece according to one of the preceding claims, **characterized by** an adapter element (20) comprising a nozzle holder receptacle (30) adapted to receive said nozzle holder (32) and a tube well (22) adapted to receive a tube (14) that is in communication with said nozzle element (44).

9. Handpiece according to claim 8, **characterized in that** said nozzle holder (32) and/or said tube are (14) is adhesively bonded and/or connected by overmolding to said adapter element (20).

10. Handpiece according to claim 9, **characterized in that** said adapter element (20) comprises an adhesive introduction opening (52) reaching up to said nozzle holder (32) and/or an adhesive introduction opening (24) reaching up to said tube (14).

11. Handpiece according to one of the claims 8 to 10, **characterized in that** a filter element (34) is arranged between said nozzle holder (32) and said tube (14).

12. Handpiece according to one of the claims 8 to 11, **characterized in that** said adapter element (20) is at its outer circumferential surface provided with a positive-fit element (80) which interacts with a positive-fit mating element (88) provided on said handpiece (2) and/or on said nozzle holder (32) for the radial positioning of said adapter element (20).

13. Insertion member for a handpiece according to one of the preceding claims with a nozzle element (44) and a unitary nozzle holder (32) which is fixedly connected to an outer circumferential surface of said nozzle element (44) and sealingly abuts against the outer circumferential surface of said nozzle element (44).

14. Insertion member according to claim 13, **characterized by** a development according to claims 2 and 3 to 7.

## Revendications

1. Pièce à main pour la pulvérisation d'un jet de liquide, comprenant un boîtier de pièce à main (2) dans lequel est logé un élément de buse (44) pour la formation de la géométrie du jet de liquide, l'élément de buse (44) étant relié de manière fixe à un support de buse (32) d'une seule pièce et étant fixé par rapport au boîtier de pièce à main (2), **caractérisée en ce que** le support de buse (32) est réalisé en matière plastique et est relié à l'élément de buse (44) par moulage par injection et s'applique de manière étanche contre la surface périphérique extérieure de l'élément de buse (44).

2. Pièce à main selon la revendication 1, **caractérisée en ce que** l'élément de buse (44) fait saillie par son extrémité de sortie au-delà du porte-buse (32) et est reçu par son extrémité d'entrée à l'intérieur du porte-buse (32).

3. Pièce à main selon l'une des revendications précédentes, **caractérisée en ce que** l'élément de buse (44) présente sur sa surface périphérique extérieure un contour de surface pour une liaison par complémentarité de forme entre le porte-buse (32) et l'élément de buse (44).

4. Pièce à main selon l'une des revendications précédentes, **caractérisée en ce qu'**un canal d'écoulement (40) formé dans le porte-buse (32) est monté devant une extrémité d'entrée de l'élément de buse (44), dont le diamètre est plus grand que le diamètre extérieur de l'élément de buse (44).

5. Pièce à main selon l'une des revendications précédentes, **caractérisée par** un capuchon de buse (58) qui s'engage sur le support de buse (32) du côté de la sortie.

6. Pièce à main selon la revendication 5, **caractérisée en ce qu'**un espace libre (56) à travers lequel passe l'élément de buse (44) est formé entre le support de buse (32) et le capuchon de buse (58).

7. Pièce à main selon l'une des revendications 5 ou 6, **caractérisée par** un joint torique (88) prévu entre le capuchon de buse (58) et le support de buse (32) et traversé par l'élément de buse (44).

8. Pièce à main selon l'une des revendications précédentes, **caractérisée par** un élément adaptateur (20), qui comporte un support de buse (30) conçu pour recevoir le support de buse (32) et un support de tuyau (22) adapté pour recevoir un tuyau (14) communiquant avec l'élément de buse (44).

9. Pièce à main selon la revendication 8, **caractérisée en ce que** le support de buse (32) et/ou le tuyau (14) est collé et/ou relié par surmoulage à l'élément adaptateur (20).

10. Pièce à main selon la revendication 9, **caractérisée en ce que** l'élément adaptateur (20) présente une ouverture d'insertion de colle (52) s'étendant jusqu'au support de buse (32) et/ou une ouverture d'insertion de colle (24) s'étendant jusqu'au tuyau (14).

11. Pièce à main selon l'une des revendications 8 à 10, **caractérisée en ce qu'**un élément filtrant (34) est disposé entre le support de buse (32) et le tuyau (14).

12. Pièce à main selon l'une des revendications 8 à 11, **caractérisée en ce que** l'élément adaptateur (20) est pourvu sur sa surface périphérique extérieure d'un élément à ajustement de forme (80) qui coopère avec un contre-élément à ajustement de forme (82) prévu sur la pièce à main (2) et/ou sur le porte-buse (32) pour le positionnement radial du corps adaptateur (20).

13. Pièce d'insertion pour une pièce à main selon l'une des revendications précédentes, comprenant un élément de buse (44) et un support de buse (32) d'une seule pièce, qui est relié de manière fixe à une surface périphérique extérieure de l'élément de buse (44), est formé en matière plastique et est relié à l'élément de buse (44) par surmoulage et s'applique de manière étanche contre la surface périphérique extérieure de l'élément de buse (44).

14. Pièce d'insertion selon la revendication 13, **caractérisée par** au moins un élément caractéristique selon les revendications 2 et 3 à 7.
